# EUROPEAN PATENT SPECIFICATION

(11) **EP 0 352 878 B1**
(45) Date of publication and mention of the grant of the patent: **23.08.1995**
(21) Application number: 89303433.0
(22) Date of filing: 07.04.1989
(51) Int. Cl.: B01J 27/10, C07C 2/86, C07C 45/46, C07C 15/16

(54) **Catalysts comprising metal compounds supported on a clay or hydrous silicate and their use**
Katalysatoren, die auf Ton oder wasserhaltiges Silikat aufgebrachte Metallverbindungen enthalten, und ihre Verwendung
Catalyseurs comprenant des composés métalliques fixés sur un support du type argile ou silicate hydraté et leur utilisation

(30) Priority: 08.04.1988 GB 8808239
(43) Date of publication of application: 31.01.1990
(60) Divisional application: 94109499.7
(73) Proprietor: CONTRACT CHEMICALS (KNOWSLEY) LIMITED, Warrington Cheshire WA1 1DL (GB)
(72) Inventor: Brown, Christopher Martin, Warrington Cheshire (GB); Barlow, Simon John, Warrington Cheshire (GB); McQuarrie, Duncan James, Winsford Cheshire (GB); Clark, James Hanley, York (GB); Kybett, Adrian Peter, Tadcaster York Yorkshire (GB)
(74) Representative: Gore, Peter Manson

(56) References cited:
- EP-A- 0 144 219
- EP-A- 0 206 265
- GB-A- 1 265 152
- HELVETICA CHIMICA ACTA, vol. 70, 1987, Basel P. LASZLO et al. "Catalysis ofFriedel-Crafts alkylation by a Montmorillonite Doped with Transition-MetalCations"pages 577-586
- CHEMICAL ABSTRACTS, vol. 108, no. 21, May 23, 1988, Columbus, Ohio, USA HASSAN,B.E.M. et al. "Catalytic alkylation of benzene in the vapor phase." page 626,abstract-no. 185 919w
- JOURNAL OF THE AMERICAN CHEMICAL SOCIETY, vol. 94, 1972 G.A.OLAH et al."Aromatic Sub-stitution. XXX. Friedel-CraftsBenzylation of Benzene and Toluenewith Benzyl and Substituted Benzyl Halides" pages 7448-7461

## Description

The present invention relates to a catalyst. It also relates to its use in alkylation reactions.

Many catalysts are known for use in alkylation of materials. Such materials may be open chain components or cyclic, and may be substituted. The materials may be based on hydrocarbon chains or may have atoms other than carbon in the backbone structure and thereby form heterocyclic rings. Particular materials which have been the subject of many publications, particularly patent specifications, include benzene. Benzene can be alkylated with benzyl chloride to form diphenylmethane.

It has however been found difficult to achieve high yields of the desired product and to have a commercially worthwhile process in that the product can be easily recovered from the reaction mixture whilst also enabling the catalyst to also be separated and re-used.

EP-A- 0037628 discloses a process for the production of a diphenylmethane in which "a" benzene is reacted with "an" α-chloromethyl benzene in the presence of sulphuric acid and a surfactant selected from particular classes. The surfactants include cationic surfactants which are stated to include phase transfer catalysts. Other surfactants which may be used are non-ionic surfactants which it is stated are believed to operate in the same manner as the cationic surfactants. When such a process was operated (as described in Example 12 thereof) using benzene, benzylchloride and 86% to 90% by weight sulphuric acid in a molar ratio of 10:1:2.5, with a phase transfer catalyst in an amount of 4% by weight with respect to the benzylchloride, and 1 hour reflux, a product is obtained in a yield of 85%. Other examples refer to substituted analogues and also to a wider range of acid strength and catalyst concentrations. However work-up appears difficult and involves dilution with water, separation, and further washing/neutralization before distillation.

US-A- 3 679 760 discloses a process for the preparation of diarylalkane which comprises reacting an α-haloalkylaromatic compound with an aromatic compound in the presence of a copper halide catalyst. In particular it refers to reacting benzene, with benzylchloride to form diphenylmethane. Whilst there is reference to reacting benzene benzylchloride and cupric chloride in a weight ratio of 1.8:1:0.33 ( a molar ratio of 3:1:0.30), there is little information on the yield or selectivity of the reaction. Indeed it is only stated that the presence of diphenylmethane was disclosed in the product by gas-liquid chromatography.

US-A- 3 678 112 is a similar disclosure to US-A- 3 679 760 but is concerned with alkylation or benzylation using an alkylic halide or a benzylic halide.

US-A- 4 251 675 is specifically directed to the preparation of a diphenylmethane compound by reacting benzene with a benzyl halide using ferric chloride as a catalyst, there being careful control of the ratios of the reactants and of the catalyst. There is only specifically disclosed a reaction of 1.2 mole of benzene with 0.4 mole of benzylchloride in the presence of 0.0002 mole of ferric chloride, the reaction being conducted at 65°C for 2.5 hours. The yield would appear to be rather low and the selectivity was only 25%. Recovery would appear to require washing out of the catalyst which would not be filterable. The catalyst presumably could therefore not be re-used.

Laszlo and Mathy (Helv. Chim. Acta., 1987, 70, 577-586) report a transition metal ion exchanged montmorillonite. The best yield in the reaction of benzylchloride with benzene was obtained with a Ti^{(IV)} exchanged montmorillonite catalyst. The fastest reaction was with an Fe^{(III)} exchanged montmorillonite catalyst. In both cases reflux conditions were required, with a 1M solution of benzylchloride in benzene and 1g of catalyst. In the former case 66% diphenylmethane was obtained in 5 hours; and 57% in the latter. In both cases significant proportions of other products were obtained.

It has now been found possible to provide a novel catalyst whose use in alkylation reactions, overcomes the problems referred to hereinabove.

According to one aspect of the present invention there is provided a catalyst, particularly an alkylation catalyst, which comprises a compound of a metal which is zinc, copper or nickel, supported on a clay, the metal compound being present in an amount of 0.1 to 20m mol per gram of clay and the catalyst being one which gives a minimum rate of conversion of 20% per hour at room temperature and atmospheric pressure of benzylchloride to diphenyl-methane in a reaction mixture comprising benzene (140 ml), benzylchloride (10 ml), and catalyst (8g at 1 m mol metal ion per gramme of catalyst),and being obtainable by a method which comprises preparing a solution of a salt of zinc, copper or nickel in an alcohol followed by mixing a clay with the solution and removing the alcohol solvent by evaporation without preliminary separation of the clay from the solvent.

The term "clay" is unusual in that it has decidely different meanings to technologists in different fields. A definition which seems to be particularly applicable to the clay supports which may be used in the present invention is that in the Penguin Dictionary of Science, namely "finely divided rock materials whose component minerals are various silicates, mainly of magnesium or aluminium". Another definition, frequently used by chemists is "a naturally occurring sediment (including that obtained by alteration in situ by supergene and hydrothermal processes) or sedimentary rock composed of one or more minerals and accessory compounds, the whole usually being rich in hydrated silicated of aluminium, iron or magnesium, hydrated alumina, or iron oxide, predominating in particles of colloidal or near-colloidal size, and commonly developing plasticity when sufficiently pulverised and wetted" (see Kirk-Othmer, Encyclopaedia of Chemical Technology, Volume 5, page 544, 2nd edition, John Wiley and Sons, Inc., New York, New York 1964).

Especially useful clays have been found to be the "montmorillonite" clays. The "montmorillonite" clays refer generally to crystalline clays having three layers. These clays are composed of units having two layers of silica tetrahedrons and one centrally disposed dioctahedral or trioctahedral layer. Such montmorillonite clays may be classified as either having an expanding lattice structure or a non-expanding lattice, (see The Encyclopaedia of Chemistry, third edition, Van Nostrand Reinhold Co., New York, 1973). Chemically, they may be considered to be clays of the general formula HAlSi₂O₆ but with variable A1-Si ratios, variable amounts of water and usually containing variable amounts of exchangeable cations.

The term "montmorillonite" has also been used to specify a species member of the "montmorillonite" genius: i.e. an aluminous member of the group accorded the empirical Formula 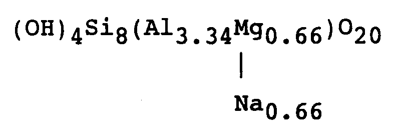
Both bentonite and hectorite are members of the expanding lattice montmorillonite subgenus. One difference between the two is that in hectorite the aluminium present in the bentonite lattice is almost completely substituted by magnesium atoms. Another difference is that lithium and fluorine are absent from the bentonite lattice and present in the hectorite lattice. Bentonite may be characterised as a sodium aluminium silicate, with hectorite being a sodium magnesium-fluor-litho silicate.

According to another aspect of the present invention there is provided a method for the production of the catalyst of the invention which comprises preparing a solution of a salt of zinc, copper or nickel in an alcohol, followed by mixing a clay with the solution, and removing the alcohol solvent by evaporation, without preliminary separation of the clay from the solvent.

The clay used in this process may be an ion exchanged clay prepared by washing or stirring a clay with a solution of a salt of zinc, copper or nickel in an alcohol solvent until no further iron is removed into the alcohol solvent, and separating the clay from the solution.

One method of preparing a catalyst of the present invention thus comprises firstly preparing a solution of a salt of zinc, copper or nickel in an alcohol solvent, followed by mixing the clay with the solution (usually by stirring the solution in the presence of the clay), and preferably then slowly evaporating the alcohol solvent.

Since the concentration of the metal compound in the solution changes during the preparation of the catalyst due to evaporation, the concentration used is not of importance. Any concentration can be used bearing in mind the amount of solid and the size of the vessel in which the catalyst is being prepared, together of course with the solubility of the particular salt. However it is preferred to use concentrations close to saturation so as to have less solvent to subsequently remove.

The compound of the metal is usually a salt. Examples of suitable salts are nitrates, phosphates, sulphates and, particularly halides. Whilst the halides may be iodides or bromides, the chlorides are preferred. Particularly useful chlorides are zinc chloride, nickel chloride, and cupric chloride. The metal may however be present in the anion rather than in the cation as in the salts particularly disclosed herein. The particularly preferred metals are zinc and nickel, especially zinc.

The solvents employed are selected from alcohols, particularly methanol. It is necessary to bear in mind the restricted solubility of the metal compounds.

Zinc chloride and clay form particularly useful catalysts. A particular catalyst (which we refer to as "clayzic") can be prepared by stirring a solution of zinc chloride in methanol containing clay in accordance with the first method as described above. The methanol is then removed by evaporation. A corresponding catalyst prepared in accordance with the second method as described above is one which we refer to as "clayziczic".

The loading of the metal compound onto the clay is usually in the range of 0.1 to 20 m mol of metal compound per gram of clay, preferably 0.2 to 2 m mol per gram, and more preferably about 1 m mol per gram.

The catalyst so produced may then be activated by heating, usually at a temperature in the range of 50°C to 300°C. A separate activation step may not be required, since when solvent is evaporated, such evaporating usually involves heating and that heating leads to activation.

According to another aspect of the present invention there is provided a process for the production of an alkylated compound which comprises reacting an organic substrate (as hereinafter defined) with an alkylating agent in the presence of a catalyst of the present invention or produced in accordance with the process of the present invention.

The catalyst is as more particularly described hereinabove, particularly with reference to its preparation.

The organic substrate may be a straight chain or branched hydrocarbon compound. However it may also be a cyclic structure which is carbocyclic or heterocyclic.

Examples of suitable organic substrates which may be used in the process of the present invention include those compounds having the general formula 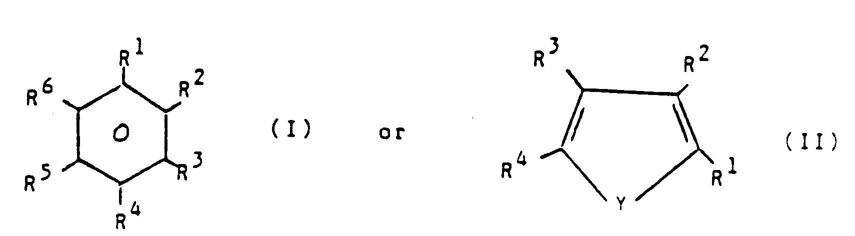
where each of R¹ to R⁴ independently is
(a) H, D (deuterium), F, Cl, Br, I;
(b) alkyl;
(c) OR;
(d) SR:
(e) NR₂
(f) COR
(g) 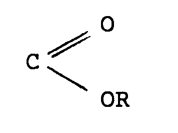
(h) 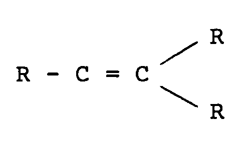
(i) C ≡ C - R
(j) CN
(k) NO₂
wherein each R independently is H, D, alkyl, or aryl which may be substituted; and Y is O, NH, ND, NR (wherein R is as defined above) or S.

Any two substituents may be joined to form a ring, which in turn may be substituted.

Suitable alkylating agents which may be used in the processes of the present invention include
(1) R⁷ R⁸ R⁹ CX¹
(2) R⁷ R⁸ C = CR⁹ R¹⁰
(3) R⁷C ≡ CR⁸
(4) R⁷ COR⁷
wherein each of R⁷ to R¹⁰ independently is as defined hereinabove as for R in respect of general formulae (I) and (II), and
X¹ is F, Cl, Br, I, o-tosyl, OH or OR (R being as hereinabove defined).

The substance to be alkylated (the substrate) is reacted with the alkylating agent in the presence of the catalyst. The molar ratio of reactants is usually (agent:substrate) in the range 1:20, preferably 1:5 to 1:10.

The amount of catalyst employed ranges widely depending on the particular catalyst and the reactants. Whilst it may be within the wide range of 0.1% to 100%, it is usually 0.1% to 10% for alkylation reactions. The percentages are based on the alkylating agent.

The temperature at which the alkylation is conducted usually depends on the particular reaction but is generally in the range of O°C to 200°C.

Whilst the alkylating agent may be added slowly to the reaction medium, it may be added totally at the commencement. The reaction medium may also contain a solvent. The solvents which may be employed include saturated hydrocarbons, aromatic hydrocarbons of lower activity than the substrate, and chlorinated hydrocarbons, such as, for example, chloroform. However the substrate itself may act as the solvent.

It is also possible to conduct the process under ambient or elevated pressure.

On completion of the reaction the catalyst may be filtered from the reaction medium and re-used. Purification and isolation of the product may be performed in a conventional manner.

The ability to recover the clay supported catalyst is an important advantage of the present invention. In comparing the present process with that disclosed in EP-A- 0037628 it has been found that whilst a similar rate of reaction occurs and the present process have a very slightly lower conversion, the working up of the product (involving filtration) is much easier. In contrast, in the process of EP-A-0037628 the phase transfer catalyst is lost as is presumably the sulphuric acid unless detailed steps are taken for the re-cycling of the acid. Futher catalysts within the scope of the present invention can be much more powerful than the phase transfer catalysts used in EP-A- 0037628. For example, zinc chloride or nickel chloride supported on clay enable complete reaction of benzene with benyl chloride to be obtained in only 15 minutes at a temperature as low as 20°C.

In connection with US-A- 3 679 760 referred to hereinabove it is believed that the mentioned ratios of reactants and catalyst give poor selectivity. Whilst it may be that in the process the catalyst, which is present in considerable amounts, could be filtered off, there is no disclosure as to the possibility of it being re-usable. It will be noted that reaction times (11 hours) and high temperatures (90°C) are employed than for the corresponding aspects of the present invention. Furthermore the present invention employs considerably less catalyst (and even that amount is re-usable) whilst producing a faster reaction.

As mentioned above, the catalysts employed in US-A- 4 256 675 presumably cannot be re-used. We have attempted to reproduce the process of that specification but problems arose in the washing stage in respect to attempted separation since emulsions formed which separated only very poorly so leading to loss of product, as well as discolouration.

The present invention will now be further described with reference to the following Examples, but is in no manner limited thereto.

### Example 1

Reaction of benzene and benzylchloride in the presence of zinc chloride supported on montmorillonite-K10 was conducted as follows. A catalyst was prepared by dissolving 10 m mol of zinc chloride in 250 ml of methanol. 10g of the clay were added, and the mixture stirred. The methanol was then removed using a rotary evaporator. The catalyst so produced (clayzic) was then heated in an oven at 170°C for 18 hours. 8g of this catalyst were then added to a solution of benzylchloride (10 ml) in benzene (140 ml), and the mixture stirred at room temperature. After 15 minutes 99% conversion of the benzylchloride to diphenylmethane was achieved. Three further additions of benzene and benzylchloride were made, with conversion in each case being in excess of 90%.

### Example 2

Reaction of benzene and benzyl chloride in the presence of copper II chloride supported on montmorillonite K10 was conducted as follows. Example 1 was repeated using the same reaction conditions but with copper (II) chloride in place of zinc chloride. After one hour, 52% conversion of benzyl chloride was achieved.

### Example 3

The procedure of Example 1 was repeated, but using zinc bromide in place of zinc chloride. After 1 hour 95% conversion of benzylchloride was achieved.

### Example 4

The procedure of Example 1 was repeated, but using zinc iodide in place of zinc chloride. After 15 minutes, 91% conversion of benzylchloride was achieved.

### Example 5

The procedure of Example 1 was repeated, but using nickel chloride in place of zinc chloride. After 15 minutes, 96% conversion of benzylchloride was achieved.

### Example 6

The procedure of Example 1 was repeated, but using zinc nitrate in place of zinc chloride. After 1 hour, 60% conversion of benzylchloride was achieved.

### Example 7

The procedure of Example 1 was repeated, but using zinc sulphate in place of zinc chloride. After 1 hour, 30% conversion of benzylchloride was achieved.

### Example 8

The procedure of Example 1 was repeated, but using zinc phosphate in place of zinc chloride. After 15 minutes, 66% conversion of benzylchloride was achieved.

### Example 9

Reaction of benzene and paraformaldehyde in the presence of zinc chloride and montmorillonite K10 was conducted as follows. A catalyst was prepared by dissolving 20 m mol of zinc chloride in 200 ml of methanol. 20g of the clay were added to this solution and the mixture is stirred for 15 minutes. The solids were then separated by filtration and added to a second zinc chloride solution (as before).

This mixture was then stirred and filtered as before. The procedure was repeated until no iron ions could be detected in the methanolic filtrate solution. 10g of the ion exchanged clay, thus prepared, were then added to a solution of 10 m mol of zinc chloride in methanol, and the solvent removed by evaporation as in Example 1. The catalyst thus prepared was referred to as clayziczic.

10g of clayziczic were added to 170 ml of benzene and 2.5g of paraformaldehyde, and the mixture was then stirred at 80°C. Three further quantities of 2.5g of paraformaldehyde were added to the reaction mixture hourly.

After 5 hours, 11.5g of diphenylmethane were produced.

### Example 10

Reaction of benzene and sec-butylchloride in the presence of clayzic (produced as in Example 1) was conducted as follows. 60g of benzene and 11g of sec-butylchloride were stirred with 1g of clayzic at 80°C. After 4 hours 12g of sec-butylbenzene were formed.

### Example 11

Reaction of benzene and benzhydrol in the presence of clayzic (produced as in Example 1) was conducted as follows. 150 ml of benzene and 15g of benzhydrol were stirred with 3g of clayzic at 80°C for 2 hours. The conversion of benzhydrol was 69% triphenylmethane, 13% diphenylmethane and 13% benzophenone.

## Claims

1. A catalyst which comprises a compound of a metal which is zinc, copper or nickel, supported on a clay, the metal compound being present in an amount of 0.1 to 20m mol per gram of clay and the catalyst being one which gives a minimum rate of conversion of 20% per hour at room temperature and atmospheric pressure of benzylchloride to diphenyl-methane in a reaction mixture comprising benzene (140 ml), benzylchloride (10 ml), and catalyst (8g at 1 m mol metal ion per gramme of catalyst),and being obtainable by a method which comprises preparing a solution of a salt of zinc, copper or nickel in an alcohol, followed by mixing a clay with the solution and removing the alcohol solvent by evaporation without preliminary separation of the clay from the solvent.

2. A catalyst according to claim 1, wherein the compound is a salt.

3. A catalyst according to claim 2, wherein the salt is a chloride.

4. A catalyst according to claim 3, wherein the chloride is zinc chloride, nickel chloride or cupric chloride.

5. A method for the production of a catalyst according to any of claims 1 to 4, which comprises preparing a solution of a salt of zinc, copper or nickel in an alcohol, followed by mixing a clay with the solution, and removing the alcohol solvent by evaporation, without preliminary separation of the clay from the solvent.

6. A method as claimed in claim 5, wherein the clay is an ion-exchanged clay prepared by washing or stirring a clay with a solution of a salt of zinc, copper or nickel in an alcohol solvent until no further iron is removed into the alcohol solvent, and separating the clay from the solution.

7. A method according to claim 5 or 6 wherein the catalyst is activated by heating, either as a separate step or as a result of heating to evaporate the alcohol solvent.

8. A method according to any of claims 5 to 7, wherein the salt is a halide.

9. A method according to claim 8, wherein the halide is a chloride.

10. A method according to any of claims 5 to 9, wherein the alcohol is methanol.

11. A process for the production of an alkylated compound which comprises reacting an organic substrate which is a straight chain or branched chain hydrocarbon or has a cyclic structure which is carbocyclic or heterocyclic with an alkylating agent in the presence of a catalyst according to any of claims 1 to 4 or as produced by a method according to any of claims 5 to 10.

12. A process according to claim 11, wherein the organic substrate is a compound having the general formula 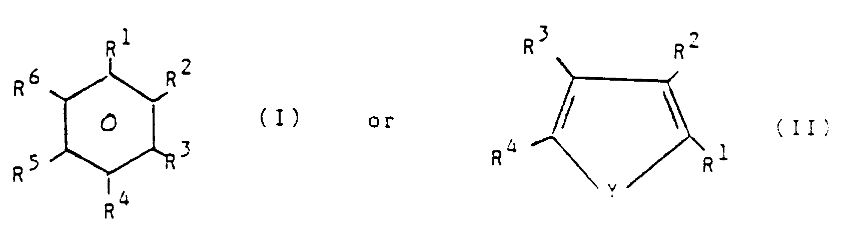 where each of R¹ to R⁴ independently is
(a) H, D (deuterium), F, Cl, Br, I;
(b) alkyl;
(c) OR;
(d) SR:
(e) NR²
(f) COR
(g) 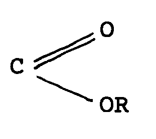
(h) 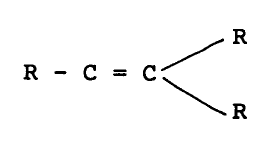
(i) C = C - R
(j) CN
(k) NO₂
wherein each R independently is H, D, alkyl, or aryl which may be substituted; and Y is O, NH, ND, NR (wherein R is as defined above) or S.

13. A process according to claim 11 or 12, wherein the alkylating agent has the general formula
(1) R⁷ R⁸ R⁹ CX¹
(2) R⁷ R⁸ C = CR⁹R¹⁰
(3) R⁷C = CR⁸
or
(4) R⁷COR⁷
wherein each of R⁷ to R¹⁰ independently is as defined as for R as defined in claim 12,
X¹ is F, C , Br, I, o-tosyl, OH or OR (R being as defined in claim 12.

## Patentansprüche

1. Katalysator, der eine Verbindung aus einem Metall umfaßt, welches Zink, Kupfer oder Nickel ist, die auf einem Ton gehalten wird, wobei die Metallverbindung in einer Menge von 0,1 bis 20 mmol pro Gramm Ton vorliegt und es sich bei dem Katalysator um einen handelt, welcher eine Mindestumwandlungsrate von 20% pro Stunde bei Raumtemperatur und atmosphärischem Druck von Benzylchlorid in Diphenylmethan in einem Reaktionsgemisch bereitstellt, das Benzol (140 ml), Benzylchlorid (10 ml) und Katalysator (8 g bei 1 mmol Metallion pro Gramm Katalysator) umfaßt und durch ein Verfahren erhaltbar ist, welches das Herstellen einer Lösung aus einem Salz von Zink, Kupfer oder Nickel in einem Alkohol, gefolgt vom Mischen eines Tons mit der Lösung und Entfernen des Alkohollösungsmittels durch Verdampfung ohne Vortrennung des Tons von dem Lösungsmittel umfaßt.

2. Katalysator nach Anspruch 1, worin die Verbindung ein Salz ist.

3. Katalysator nach Anspruch 2, worin das Salz ein Chlorid ist.

4. Katalysator nach Anspruch 3, worin das Chlorid Zinkchlorid, Nickelchlorid oder Kupfer(II)-chlorid ist.

5. Verfahren für die Herstellung eines Katalysators nach einem der Ansprüche 1 bis 4, welches das Herstellen einer Lösung aus einem Salz von Zink, Kupfer oder Nickel in einem Alkohol umfaßt, gefolgt vom Mischen eines Tons mit der Lösung und Entfernen des Alkohollösungsmittels durch Verdampfung ohne Vortrennung des Tons von dem Lösungsmittel.

6. Verfahren nach Anspruch 5, worin der Ton ein ionenausgetauschter Ton ist, der durch Waschen oder Rühren eines Tons mit einer Lösung aus einem Salz von Zink, Kupfer oder Nickel in einem Alkohollösungsmittel hergestellt wird, bis kein weiteres Eisen mehr in das Alkohollösungsmittel entfernt wird und Trennen des Tons von der Lösung.

7. Verfahren nach Anspruch 5 oder 6, worin der Katalysator durch Erwärmen aktiviert wird, entweder als ein getrennter Schritt oder aufgrund von Erwärmen zum Verdampfen des Alkohollösungsmittels.

8. Verfahren nach einem der Ansprüche 5 bis 7, worin das Salz ein Halogenid ist.

9. Verfahren nach Anspruch 8, worin das Halogenid ein Chlorid ist.

10. Verfahren nach einem der Ansprüche 5 bis 9, worin der Alkohol Methanol ist.

11. Prozeß für die Herstellung einer alkylierten Verbindung, welcher das Reagieren eines organischen Substrates umfaßt, bei dem es sich um einen geradkettigen oder verzweigtkettigen Kohlenwasserstoff handelt oder das eine zyklische Struktur besitzt, welche karbozyklisch oder heterozyklisch ist, mit einem alkylierenden Mittel in Anwesenheit eines Katalysators nach einem der Ansprüche 1 bis 4 oder wie anhand eines Verfahrens nach einem der Ansprüche 5 bis 10 hergestellt.

12. Prozeß nach Anspruch 11, worin das organische Substrat eine Verbindung mit der folgenden allgemeinen Formel ist: 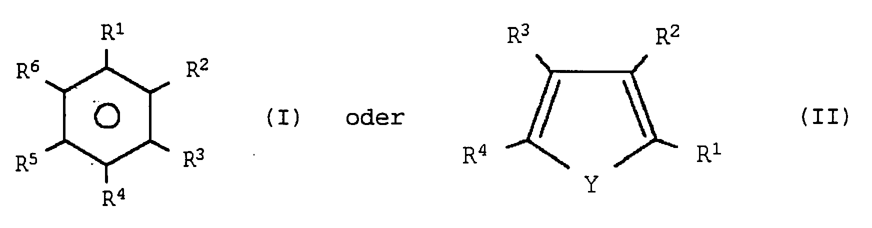 worin jeweils jedes R¹ bis R⁴ unabhängig folgendes ist:
(a) H, D (Deuterium), F, Cl, Br, I;
(b) Alkyl;
(c) OR;
(d) SR:
(e) NR²
(f) COR
(g) 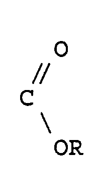
(h) 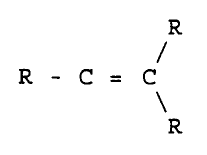
(i) C = C - R
(j) CN
(k) NO₂
worin jedes R unabhängig H, D, Alkyl oder Aryl ist, welches substituiert werden kann; und Y O, NH, ND, NR (worin R wie oben definiert ist) oder S ist.

13. Prozeß nach Anspruch 11 oder 12, worin das alkylierende Mittel die allgemeine Formel
(1) R⁷ R⁸ R⁹ CX¹
(2) R⁷ R⁸ C = CR⁹R¹⁰
(3) R⁷C = CR⁸
oder
(4) R⁷COR⁷
besitzt, worin jeweils jedes R⁷ bis R¹⁰ unabhängig auf die gleiche Weise wie R nach Anspruch 12 definiert ist
X¹ F, C, Br, I, o-Tosyl, OH oder OR ist (wobei R wie nach Anspruch 12 definiert ist).

## Revendications

1. Catalyseur qui comporte un composé d'un métal qui est du zinc, du cuivre ou du nickel, fixé sur un support d'argile, le composé métallique étant présent dans une quantité de 0,1 à 20 millimoles par gramme d'argile et le catalyseur donnant un taux minimum de conversion de 20% par heure, à température ambiante et sous pression atmosphérique, de chlorure de benzyle en diphénylméthane dans un mélange réactionnel comportant du benzène (140ml), du chlorure de benzyle (10ml), et un catalyseur (8g à un millimole d'ion métallique par gramme de catalyseur), et pouvant être obtenu par un procédé qui comprend la préparation d'une solution d'un sel de zinc, de cuivre ou de nickel, dans un alcool, suivi du mélange d'un argile avec la solution et de l'enlèvement du solvant à l'alcool par évaporation sans séparation préliminaire de l'argile du solvant.

2. Catalyseur selon la revendication 1, dans lequel le composé est un sel.

3. Catalyseur selon la revendication 2, dans lequel le sel est un chlorure.

4. Catalyseur selon la revendication 3, dans lequel le chlorure est du chlorure de zinc, du chlorure de nickel ou du chlorure cuivrique.

5. Procédé pour la production d'un catalyseur selon l'une quelconque des revendications 1 à 4, qui comprend la préparation d'une solution d'un sel de zinc, de cuivre ou de nickel dans un alcool, suivi du mélange d'un argile avec la solution, et de l'enlèvement du solvant à l'alcool par évaporation, sans séparation préliminaire de l'argile du solvant.

6. Procédé selon la revendication 5, dans lequel l'argile est un argile d'échange d'ions préparé en lavant ou en mélangeant un argile avec une solution d'un sel de zinc, de cuivre ou de nickel dans un solvant à l'alcool, jusqu'à absence de fer dans le solvant à l'alcool, et en séparant l'argile de la solution.

7. Procédé selon la revendication 5 ou 6, dans lequel le catalyseur est activé par chauffage, soit en tant qu'étape séparée ou soit en tant que résultat du chauffage pour évaporer le solvant à l'alcool.

8. Procédé selon l'une quelconque des revendications 5 à 7, dans lequel le sel est un halogénure.

9. Procédé selon la revendication 8, dans lequel l'haloïde est un chlorure.

10. Procédé selon l'une quelconque des revendications 5 à 9, dans lequel l'alcool est du méthanol.

11. Procédé pour la production d'un composé alcoylé qui comporte la réaction d'un substrat organique qui soit un carbure d'hydrogène à chaîne linéaire ou à chaîne ramifiée ou qui présente une structure cyclique qui soit homocyclique ou hétérocyclique avec un agent d'alcoylation en présence d'un catalyseur selon l'une quelconque des revendications 1 à 4 ou comme réalisé par un procédé selon l'une quelconque des revendications 5 à 10.

12. Procédé selon la revendication 11, dans lequel le substrat organique est un composé ayant la formule générale suivante 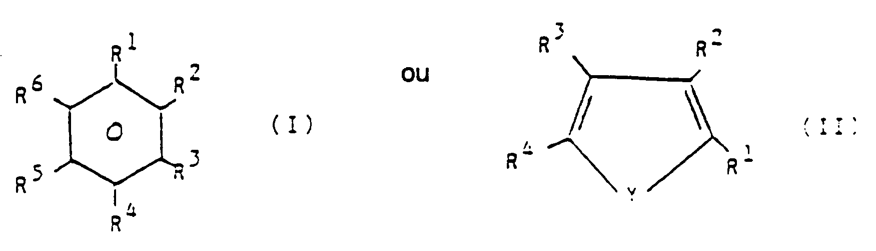 où chacun des R¹ à R⁴ est de façon indépendante
a) H, D (deutérium), F, Cl, Br, I;
b) alcoyle;
c) OR;
d) SR:
e) NR²
f) COR
g) 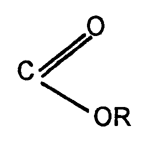
h) 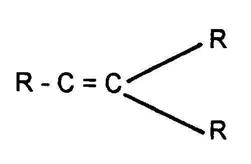
i) C = C - R
j) CN
k) NO₂
dans lequel chaque R est de façon indépendante H, D, alcoyle, ou aryle qui peut être substitué; et Y est O, NH, ND, NR (où R est défini comme ci-dessus) ou S.

13. Procédé selon la revendication 11 ou 12, dans lequel l'agent d'alcoylation a la formule général suivante
1) R⁷ R⁸ R⁹ CX¹
2) R⁷ R⁸ C = CR⁹R¹⁰
3) R⁷C = CR⁸
ou
4) R⁷COR⁷
où chacun des R⁷ à R¹⁰ de façon indépendante est comme défini pour R, comme défini dans la revendication 12,
X¹ est F, C, Br, I, o-tosyle, OH ou OR (R étant comme défini dans la revendication 12).
